# FASCICULE DE BREVET EUROPEEN

(11) **EP 1 648 513 B1**
(45) Date de publication et mention de la délivrance du brevet: **24.09.2014**
(21) Numéro de dépôt: 04786018.4
(22) Date de dépôt: 30.07.2004
(51) Int. Cl.: A61K 39/395, A61P 37/02, A61P 35/00

(54) **UTILISATION D'ANTICORPS AYANT UNE ADCC OPTIMISÉE POUR TRAITER LES PATIENTS RÉPONDANT FAIBLEMENT AU TRAITEMENT PAR ANTOCORPS.**
VERWENDUNG VON ADCC-OPTIMIERTEN ANTIKÖRPERN ZUR BEHANDLUNG VON PATIENTEN, DIE NUR SCHWACH AUF ANTIKÖRPER ANSPRECHEN.
USE OF ADCC-OPTIMIZED ANTIBODIES FOR TREATING PATIENTS ANSWERING WEAKLY TO ANTIBODY TREATMENT.

(30) Priorité: 31.07.2003 FR 0309440
(43) Date de publication de la demande: 26.04.2006
(60) Demande divisionnaire: 10181317.8
(73) Titulaire: Laboratoire Français du Fractionnement et des Biotechnologies, 91940 Les Ulis (FR)
(72) Inventeur: BOUREL, Dominique, F-59110 La Madeleine (FR); JORIEUX, Sylvie, F-59650 Villeneuve d'Ascq (FR); DE ROMEUF, Christophe, F-59000 Lille (FR); KLEIN, Philippe, 59000- Lille (FR); GAUCHET, Christine, 59320- Sédequin (FR); BIHOREAU, Nicolas, F-91400 Orsay (FR); GAUCHET, Christine, 59320 Sequedin (FR)
(74) Mandataire: Regimbeau
(86) Numéro de dépôt international: PCT/FR2004/002057
(87) Numéro de publication internationale: WO 2005/012358

(56) Documents cités:
- EP-A1- 0 576 093
- EP-A1- 1 176 195
- WO-A2-01/77181
- KUMPEL B M ET AL: "Clearance of red cells by monoclonal IgG3 anti-D in vivo is affected by the VF polymorphism of FcgammaRIIIa (CD16)." CLINICAL AND EXPERIMENTAL IMMUNOLOGY, vol. 132, no. 1, avril 2003 (2003-04), pages 81-86, XP001193742 ISSN: 0009-9104 cité dans la demande
- SHINKAWA T ET AL: "The absence of fucose but not the presence of galactose or bisecting N-acetylglucosamine of human IgG1 complex-type oligosaccharides shows the critical role of enhancing antibody-dependent cellular cytotoxicity" JOURNAL OF BIOLOGICAL CHEMISTRY, AMERICAN SOCIETY OF BIOLOGICAL CHEMISTS, BALTIMORE, MD, US, vol. 278, no. 5, 31 janvier 2003 (2003-01-31), pages 3466-3473, XP002965857 ISSN: 0021-9258 cité dans la demande
- UMANA P ET AL: "ENGINEERED GLYCOFORMS OF AN ANTINEUROBLASTOMA IGG1 WITH OPTIMIZED ANTIBODY-DEPENDENT CELLULAR CYTOTOXIC ACTIVITY" NATURE BIOTECHNOLOGY, NATURE PUBLISHING, US, vol. 17, février 1999 (1999-02), pages 176-180, XP002921620 ISSN: 1087-0156 cité dans la demande
- SHIELDS R L ET AL: "Lack of fucose on human IgG1 N-linked oligosaccharide improves binding to human FcgammaRIII and antibody-dependent cellular toxicity" JOURNAL OF BIOLOGICAL CHEMISTRY, AMERICAN SOCIETY OF BIOLOGICAL CHEMISTS, BALTIMORE, MD, US, vol. 277, no. 30, 26 juillet 2002 (2002-07-26), pages 26733-26740, XP002964542 ISSN: 0021-9258 cité dans la demande
- CARTRON GUILLAUME ET AL: "Therapeutic activity of humanized anti-CD20 monoclonal antibody and polymorphism in IgG Fc receptor FcgammaRIIIa gene" BLOOD, vol. 98, no. 11 Part 1, 16 novembre 2001 (2001-11-16), page 602a, XP001193741 & 43RD ANNUAL MEETING OF THE AMERICAN SOCIETY OF HEMATOLOGY, PART 1; ORLANDO, FLORIDA, USA; DECEMBER 07-11, 2001 ISSN: 0006-4971 cité dans la demande
- CROWE J S ET AL: "HUMANIZED MONOCLONAL ANTIBODY CAMPATH-1H MYELOMA CELL EXPRESSION OF GENOMIC CONSTRUCTS NUCLEOTIDE SEQUENCE OF CDNA CONSTRUCTS AND COMPARISON OF EFFECTOR MECHANISMS OF MYELOMA AND CHINESE HAMSTER OVARY CELL-DERIVED MATERIAL" CLINICAL AND EXPERIMENTAL IMMUNOLOGY, OXFORD, GB, vol. 87, no. 1, 1992, pages 105-110, XP008034008 ISSN: 0009-9104
- LIFELY M R (REPRINT) ET AL: "Glycosylation and biological-activity of CAMPATH-1H expressed in different cell-lines and grown under different culture conditions" GLYCOBIOLOGY, IRL PRESS,, GB, vol. 5, no. 8, décembre 1995 (1995-12), pages 813-822, XP002096118 ISSN: 0959-6658
- N. I. OLOVNIKOVA ET AL: 'Effect of Producer Cell Line on Functional Activity of Anti-D Monoclonal Antibodies Destined for Prevention of Rhesus Sensitization' BULLETIN OF EXPERIMENTAL BIOLOGY AND MEDICINE vol. 147, no. 4, 01 Avril 2009, pages 448 - 452, XP055000368 DOI: 10.1007/s10517-009-0516-0 ISSN: 0007-4888
- SHIELDS R L ET AL: "Lack of fucose on human IgG1 N-linked oligosaccharide improves binding to human FcgammaRIII and antibody-dependent cellular toxicity", JOURNAL OF BIOLOGICAL CHEMISTRY, AMERICAN SOCIETY FOR BIOCHEMISTRY AND MOLECULAR BIOLOGY, US, vol. 277, no. 30, 26 July 2002 (2002-07-26), pages 26733-26740, XP002964542, ISSN: 0021-9258, DOI: 10.1074/JBC.M202069200
- GUILE G ET AL: "Rapid High-Resolution High-Performance Liquid Chromatographic Method for Separating Glycan Mixtures and Analyzing Oligosaccharide Profiles", ANALYTICAL BIOCHEMISTRY, vol. 240, 1996, pages 210-226,
- DWEK R.A ET AL: "Glycobiology: "The function of sugar in the IgG molecule"", J. ANAT., vol. 187, 1995, pages 279-292,
- HOLLAND M. ET AL: "Hypogalactosylation of serum IgG in patients with ANCA-associated systemic vasculitis", CLIN EXP IMMUNOL, vol. 129, 2002, pages 183-190,
- CHANG K. H. ET AL.: "Quantitative analysis of oligosaccharide structure of glycoproteins", BIOTECHNOL. BIOPROCESS ENG., vol. 5, 2000, pages 136-140,
- CHARLWOOD J. ET AL: "Characterization of the lycosylation profiles of alzheimer's beta-secretase protein Asp-2 expressed in a variety of cell lines", THE JOURNAL OF BIOLOGICAL CHEMISTRY, vol. 276, no. 20, 2001, pages 16739-16748,

## Description

La présente **description** concerne l'utilisation d'anticorps monoclonaux chimériques, humanisés ou humains de glycosylation spécifique présentant une forte interaction pour le récepteur CD16 (FcgammaRIII) des cellules effectrices du système immunitaire mais également la propriété d'induire la sécrétion de cytokines et d'interleukines, pour traiter des populations de patients répondant faiblement aux traitements existants et présentant un polymorphisme particulier de leur CD 16.

L'immunothérapie au moyen d'anticorps monoclonaux est en passe de devenir une des stratégies les plus importantes et novatrices de la médecine. Cependant, les résultats obtenus lors d'essais cliniques apparaissent contrastés. En effet, au cours d'un certain nombre de traitements, l'anticorps monoclonal n'apparaît pas assez actif et/ou efficace. De nombreux essais cliniques sont arrêtés en raison d'un manque d'efficacité et d'effets secondaires incompatibles avec une utilisation en thérapie clinique. Ces deux aspects sont étroitement liés sachant que des anticorps peu actifs et/ou efficaces sont administrés à fortes doses pour compenser le manque d'efficacité et obtenir une réponse thérapeutique. L'administration de fortes doses induit alors des effets secondaires. De plus elle est économiquement peu viable.

Ces problèmes sont majeurs dans l'industrie des anticorps monoclonaux, en particulier les anticorps chimériques, humanisés ou humains. Or, ces problèmes sont exacerbés pour des populations particulières de patients pour lesquels l'effet thérapeutique des anticorps s'avère significativement plus faible comparé à la moyenne de la population traitée. Ces patients sont communément appelés « patients faibles répondeurs ».

Il est connu que la cytotoxicité cellulaire médiée par les anticorps nécessite d'une part une fixation de la partie Fab des anticorps sur leur cible ainsi qu'un engagement de leur domaine Fc sur les récepteurs Fc des cellules effectrices (RFc). Sur les cellules NK, le récepteur responsable de cette activité cytotoxique est le CD 16.
Sur la base de ces connaissances, des études ont été menées afin d'établir une corrélation entre les sous-populations de patients réfractaires (patients dits « faibles répondeurs ») à la thérapie avec un anticorps spécifique d'une pathologie à traiter et le polymorphisme du CD 16.

Dans une étude de 2000 (Cartron G, Dacheux L, Salles G, Solal-Celigny P, Bardos P, Colombat P, Watier H, Therapeutic activity of humanized anti-CD20 monoclonal antibody and polymorphism in IgG Fc receptor FcgammaRIIIa gene, Blood. 2002 Feb 1 ;99(3):754-8), la composition de la population de patients traités avec Rituxan® a été analysée en fonction du polymorphisme du CD16 en positon 158. Celle-ci était composée de 20% de FCGR3A-158V homozygotes (patients homozygotes pour la valine en position 158 du CD16), 35% de FCGR3A-158F homozygotes (patients homozygotes pour la phénylalanine en position 158 du CD16) et de 45% d'hétérozygotes FCGR3A-158V/F (patients hétérozygotes valine/phénylalanine en position 158 du CD16). L'activité ADCC ainsi que l'efficacité clinique de l'anticorps anti-CD20 Rituxan® sont corrélées avec le polymorphisme de CD16: les patients FCGR3A-158F homozygotes ou hétérozygotes FCGR3A-158V/F sont moins bons répondeurs au traitement avec Rituxan® que les patients FCGR3A-158V homozygotes.
Ainsi, cette étude a permis de démontrer que les différences de réponse aux traitements avec des anticorps thérapeutiques sont associées à un polymorphisme du CD16. Toutefois, cette étude ne propose pas de solution pour diminuer l'effet de ce polymorphisme chez les patients dits « faibles répondeurs » traités avec des anticorps thérapeutiques. Elle ne fournit pas d'outil thérapeutique adapté au traitement des patients faibles répondeurs.

Par ailleurs, d'autres résultats ont été obtenus au termes d'études cliniques réalisées avec des anticorps anti-Rhésus, qui ont montré que l'élimination des hématies Rhésus positif par une IgG3 anti-D était plus rapide chez les patients FCGR3A-158F homozygotes (Kumpel BM, De Haas M, Koene HR, Van De Winkel JG, Goodrick MJ. Clearance of red cells by monoclonal IgG3 anti-D in vivo is affected by the VF polymorphism of Fcgamma RIIIa (CD16). Clin Exp Immunol. 2003 Apr;132(1):81-6). Toutefois, le faible nombre de patients étudiés ne permet pas de tirer un enseignement technique de cette étude.

Le but de la présente invention est de fournir des anticorps efficaces pour traiter les patients se trouvant en échec thérapeutique avec les anticorps actuellement disponibles ou subissant des effets secondaires indésirables.

La demanderesse a trouvé que des anticorps dont le domaine Fc présente une structure glycannique particulière présentent une activité cytotoxique particulièrement adaptée au traitement des patients dits « faibles répondeurs », qui n'est pas affectée par le polymorphisme FCGR3A-158F homozygotes ou FCGR3A-158V/F de l'acide aminé 158 du CD16, contrairement aux anticorps produits dans CHO dont l'activité cytotoxique est affectée par ce polymorphisme. De plus, la demanderesse a trouvé que ces anticorps induisent une production de cytokines/chémokines, ce qui participe encore au renforcement de l'effet thérapeutique en stimulant des mécanismes effecteurs du système immunitaire chez les patients traités.

L'invention propose donc d'utiliser ces anticorps pour le traitement de sous-populations de patients dits « faibles répondeurs ». Ces anticorps présentent une activité ADCC et une production de cytokine et/ou chemokines jusqu'à 100 fois supérieure aux anticorps disponibles en thérapie.

### Description

Ainsi, l'invention se rapporte **aux objets des revendications 1 à 20**.

**La présente description décrit également** l'utilisation d'un anticorps monoclonal chimérique, humanisé ou humain optimisé caractérisé en ce que :
a) il est produit dans une lignée cellulaire sélectionnée pour ses propriétés de glycosylation de la région Fc d'un anticorps, ou
b) la structure glycannique de la région Fc a été modifiée ex vivo, et/ou
c) sa séquence primaire a été modifiée de façon à augmenter son interaction vis à vis des récepteurs Fc;
ledit anticorps présentant i) un taux ADCC via le CD16 (FcgammaRIIIA) supérieur à 60 %, 70%, 80 % ou de préférence supérieur à 90 % comparé au même anticorps produit dans une lignée CHO ou à un anticorps homologue disponible dans le commerce, pour la préparation d'un médicament destiné au traitement de pathologies chez des patients présentant le polymorphisme du récepteur CD16 FCGR3A-158V/F ou FCGR3A-158F homozygotes et qualifiés de "faibles répondeurs" aux thérapies avec les anticorps actuellement disponibles.

On entend par patients « faibles répondeurs » les patients présentant une réduction de 20 à 50% de la réponse aux traitements avec des anticorps thérapeutiques par rapport aux patients dits « forts répondeurs », c'est-à-dire aux patients présentant une réponse complète correspondant à la disparition de tous les symptômes et signes cliniques mesurables de la maladie.

Par exemple, dans le cas d'une étude de clairance des hématies ou d'un autre type cellulaire dans la circulation, on entend par patients "faibles répondeurs", les patients qui présentent une clairance significativement plus longue, par rapport à un autre groupe de patients. Dans le traitement des leucémies, on distingue :
* les forts répondeurs avec une réponse complète correspondant à la disparition de tous les symptômes et des signes mesurables de la maladie, tant du point de vue de l'examen clinique que des données biologiques de laboratoires et des études radiographiques. La diminution de la taille des plus grandes tumeurs est supérieure à 75%.
* les patients qui répondent partiellement sont décrits dans l'article Cheson BD et al, Report of an international workshop to standardize response criteria for non-Hodgkin's lymphomas. NCI Sponsored International Working Group. J Clin Oncol. 1999 Apr;17(4):1244. Review. Erratum in: J Clin Oncol 2000 Jun;18(11):2351.
* les faibles répondeurs, correspondent à des patients ayant un état dit stable, avec moins de 50% de réduction de la masse tumorale, moins de 25% d'augmentation des lésions et pas de nouvelles lésions. Ce groupe de patients comprend également les patients pour lesquels aucune réponse n'est observée (progression de la maladie pouvant aller jusque la mort).

Ainsi, pour une sous population de patients dits « faibles répondeurs » en rapport avec le polymorphisme de l'acide aminé 158 du CD16 (FCGR3A-158F homozygotes ou FCGR3A-158V/F) ou un autre polymorphisme associé à ce polymorphisme du CD16, l'efficacité du traitement est meilleure avec les anticorps optimisés de l'invention, et se rapproche de celle des patients dit « forts répondeurs ».

En effet, l'interaction du récepteur pour le Fc des anticorps est différente suivant les polymorphismes du CD16 (aa 158), le phénotype FCGR3A-158V homozygote ayant une meilleure affinité que la forme FCGR3A-158F homozygote et FCGR3A-158V/F hétérozygote. Les anticorps utilisés dans le cadre de l'invention présentent une forte interaction avec le CD16, ce qui peut expliquer le fait que leur activité fonctionnelle ne soit pas ou très peu affectée par le polymorphisme FCGR3A-158F homozygotes ou FCGR3A-158V/F du CD16.

L'invention s'adresse donc à une population particulière de patients présentant un polymorphisme du CD16 (FCGR3A-158F homozygotes ou FCGR3A-158V/F), notamment des patients se trouvant en échec thérapeutique avec les anticorps actuellement disponibles et/ou subissant des effets secondaires indésirables justifiant l'administration de l'anticorps optimisé **décrit ici**.

Les pathologies traitées **dans le cadre de** l'invention ne sont pas limitées à des pathologies particulières, mais s'entendent de toutes les pathologies susceptibles d'être traitées avec des anticorps monoclonaux.

Outre l'amélioration très significative de l'activité ADCC de type CD16 (FcγRIIIA) , l'anticorps **utilisé dans le cadre** de l'invention peut être défini en ce qu'il induit la sécrétion d'au moins une cytokine par un type de cellules effectrices du système immunitaire exprimant le récepteur CD16 supérieur à 50 %, 100 % ou de préférence supérieur à 200 % comparé au même anticorps produit dans une lignée CHO ou comparé à un anticorps homologue disponible dans le commerce. Le type de cytokine est choisi parmi IL-1, IL-4, IL-12, IL-18, IL-21, IL-2, IL-3, IL-5, IL-6, IL-7, IL-8, IL-9, IL-10, IFNα, IFNβ, TNFα, TGFβ, IP10 et TNF, IFNγ.

Dans le cadre de **la présente description**, nous montrons que des anticorps présentant une forte interaction avec le CD16 ont l'avantage d'induire la production de cytokines, ou de chémokines notamment la production d' IFNγ. Les deux caractéristiques précitées se complètent. En effet, la production d'IFNγ ou d'autres cytokines ou chémokines, par des cellules effectrices, induite par les anticorps selon l'invention, peut renforcer l'effet thérapeutique en stimulant des mécanismes effecteurs du système immunitaire chez les patients traités. Le mécanisme d'action d'une telle stimulation correspond notamment à une régulation positive autocrine des cellules effectrices. Les anticorps se liant au CD16 induisent une activité cytotoxique ainsi que la production d'IFNγ ou d'autres cytokines/chémokines ce qui « in fine » conduit à augmenter encore davantage l'activité cytotoxique.

De préférence, cet anticorps présente un taux ADCC supérieur d'au moins 100 % comparé au même anticorps produit dans une lignée CHO ou à un anticorps homologue disponible dans le commerce et un taux de production d'au moins une cytokine par un type de cellules effectrices du système immunitaire exprimant le récepteur CD16 supérieur d'au moins 100 % comparé au même anticorps produit dans une lignée CHO ou à un anticorps homologue disponible dans le commerce.

Lesdites cytokines dont la libération est induite par les anticorps optimisés sont choisies parmi des interleukines, des cytokines, des chémokines, des interférons et des facteurs de nécrose tumorale (TNF).

Ainsi, l'anticorps **utilisé dans le cadre de** l'invention est capable d'induire la sécrétion d'au moins un type de cytokine choisi parmi IL-1, IL-4, IL-12, IL-18, IL-21, IL-2, IL-3, IL-5, IL-6, IL-7, IL-8, IL-9, IL-10, IFNα, IFNβ, TNFα, TGFβ, IP10 et TNF, IFNγ, par les cellules effectrices du système immunitaire.

De préférence, l'anticorps sélectionné présente la capacité d'induire la sécrétion d'IFNγ ou d'autres cytokines/chémokines par les cellules effectrices du système immunitaire exprimant le récepteur CD16 ou plus spécifiquement de l'IL2 par la cellule Jurkat CD16. Le taux d'IFNγ ou d'autres cytokines et/ou chémokines sécrétés traduit la capacité d'interaction de la région Fc de l'anticorps avec le CD16 et également traduit sa capacité de liaison à l'antigène. La sécrétion d'IFNγ ou d'autres cytokines et/ou chémokines par les cellules du système immunitaire peut accroître et/ou induire l'activité cytotoxique des cellules effectrices (NK, monocytes, macrophage, polynucléaires neutrophiles...).
Les cellules effectrices peuvent exprimer un CD16 endogène éventuellement modulable par des cytokines et/ou chémokines ou facteurs de croissance ou être transformées. On entend par cellule transformée, une cellule modifiée génétiquement de sorte à exprimer un récepteur, en particulier le récepteur CD16.

Dans un mode de réalisation particulier, l'anticorps utilisé dans le cadre de l'invention est capable d'induire la sécrétion d'au moins une cytokine par une cellule leucocytaire, en particulier de la famille des cellules NK (Natural Killer) ou par des cellules de la lignée myélo-monocytaire (monocytes-macrophages et cellule dendritique).
De préférence, on utilise pour la sélection des anticorps une lignée Jurkat transfectée avec un vecteur d'expression codant le récepteur CD16 comme cellule effectrice. Cette lignée est particulièrement avantageuse car elle est immortalisée et se maintient en culture indéfiniment. Le taux d'IL2 sécrétée traduit la capacité d'interaction de la région Fc de l'anticorps avec le CD16 et également traduit sa capacité de liaison à l'antigène.
Différentes lignées Jurkat transfectées avec un vecteur d'expression codant pour le récepteur CD16 peuvent être utilisées comme cellule effectrice, lesdites lignées exprimant chacune un CD 16 particulier.

En outre, l'anticorps optimisé peut être préparé après avoir été purifié et/ou modifié *ex vivo* en ce qui concerne la structure glycannique de sa région Fc. A cet effet, on peut utiliser tout moyen chimique, chromatographique ou enzymatique approprié pour modifier la structure glycannique de l'anticorps. Par exemple, on peut purifier des anticorps obtenus de diverses sources et ajouter dans un mélange réactionnel une ou plusieurs glycosyl transférase(s), notamment une galactosyl-transférase, incuber pendant un temps déterminé jusqu'à l'obtention des anticorps présentant la structure glycannique décrite plus haut.

La sélection peut se faire sur des anticorps produits par des cellules couramment utilisées pour la production d'anticorps thérapeutiques, telles que les lignées de myélomes de rat, en particulier YB2/0 et ses dérivés, les cellules lymphoblastoïdes humaines, les cellules d'insectes, les cellules de myélomes murins, les hybridomes ainsi que les cellules eucaryotes comme par exemple les levures.
Il est également possible de produire les anticorps dans une lignée de cellules de mammifères modifiée génétiquement, par exemple CHO modifiée génétiquement, par introduction d'une ou plusieurs séquence(s) exprimant une ou plusieurs glycosyl transférase(s), notamment sélectionnées parmi un enzyme impliqué dans la modification des chaînes oligosaccaridiques en position 1 du fucose lié en alpha à la position 6 du résidue N-acetylglucosamine à l'extrémité réductrice, en particulier une 1,6-fucosyltransférase, et une galactosyl-transférase. La sélection peut également être appliquée à l'évaluation d'anticorps produits par des plantes transgéniques ou des mammifères transgéniques.

A cet effet, la production dans CHO sert de référence (CHO étant employée pour la production d'anticorps médicament) pour comparer et sélectionner les systèmes de production conduisant aux anticorps utilisés dans le cadre de l'invention. Une comparaison avec des anticorps polyclonaux peut également être utile lors des tests d'efficacité des anticorps monoclonaux. Ainsi, pour une sous population de patients dits « faibles répondeurs » en rapport avec le polymorphisme de l'acide aminé 158 du CD16 ou un autre polymorphisme associé à ce polymorphisme, l'efficacité du traitement est meilleure avec les anticorps optimisés **utilisés dans le cadre** de l'invention, et se rapproche de celle des patients dit « forts répondeurs ». Nous montrons que l'activité fonctionnelle des anticorps monoclonaux optimisés s'apparente à celle des anticorps polyclonaux thérapeutiques. Ainsi, dans certains essais thérapeutiques, les anticorps polyclonaux peuvent être utilisés comme contrôles lors de tests d'efficacité d'anticorps monoclonaux de différentes origines. Ceci permet de sélectionner des anticorps monoclonaux destinés au traitement de sous populations de patients faiblement répondeurs.

Ainsi, dans certains essais thérapeutiques, les anticorps polyclonaux peuvent être utilisés comme contrôles lors de tests d'efficacité d'anticorps monoclonaux de différentes origines. Ceci permet de sélectionner des anticorps monoclonaux destinés au traitement de sous populations de patients faiblement répondeurs. Une autre alternative consiste à effectuer la comparaison avec les anticorps disponibles dans le commerce, en particulier les anticorps en cours de développement, les anticorps ayant obtenu une AMM ou encore des anticorps dont les essais cliniques ont été arrêtés et qui se sont révélés peu efficaces et/ou produisant des effets secondaires indésirables aux doses administrées. En effet, les anticorps modifiés utilisés dans le cadre de l'invention sont au moins 100% plus efficaces pour activer l'ADCC supportée par des cellules effectrices du système immunitaire, ce qui implique des doses d'administration inférieures à celles pratiquées par les anticorps mentionnés précédemment et dans le cas présent la possibilité de traiter des patients pour lesquels les anticorps actuellement disponibles se sont révélés inefficaces.

L'anticorps peut dans un premier temps être sélectionné pour sa capacité d'interaction avec le récepteur CD16 puis testé et sélectionné tel que décrit ci-dessus pour ses propriétés à induire la production d'une cytokine, notamment l'IL-2, par les cellules Jurkat CD16 ou d'IFNγ par les cellules effectrices exprimant le CD 16.

De tels anticorps possédant cette double propriété d'induire l'ADCC via le CD16 et d'induire la production d' IFNγ ou d'autres cytokines et/ou chémokines par les cellules du système immunitaire peut accroître et/ou induire l'activité cytotoxique des cellules effectrices (NK, monocytes, macrophage, polynucléaires neutrophiles notamment).

Ainsi, **la présente description** décrit l'utilisation de l'anticorps défini ci-dessus pour la préparation d'un médicament destiné au traitement de pathologies chez des patients homozygotes pour la phénylalanine en position 158 du CD 16 (FCGR3A-158F homozygotes) ou des patients hétérozygotes valine/phénylalanine en position 158 du CD16 (FCGR3A-158V/F).
De tels anticorps possèdent une glycosylation particulière. Ils possèdent un domaine Fc de type biantenné, avec des chaînes courtes, une faible sialylation, des mannoses et GlcNAc du point d'attache terminaux non intercalaires et une faible fucosylation.

Ainsi, dans un autre aspect, la présente description décrit l'utilisation d'un anticorps monoclonal chimérique, humanisé ou humain dont la structure glycannique du domaine Fc de l'anticorps correspond à un type biantenné, avec des chaînes courtes, une faible sialylation, des mannoses et GlcNAc du point d'attache terminaux non intercalaires, et une faible fucosylation pour la préparation d'un médicament destiné au traitement de pathologies chez des patients homozygotes pour la phénylalanine en position 158 du CD16 (FCGR3A-158F homozygotes) ou des patients hétérozygotes valine/phénylalanine en position 158 du CD16 (FCGR3A-158V/F).

En effet, cette forme glycannique du domaine Fc de l'anticorps confère à celui-ci les propriétés d'induire une forte ADCC et la production de cytokines et/ou chémokines tel que décrit précédemment.
Dans cet anticorps, le taux de GlcNac intercalaire est non nul.
Dans le cadre de l'invention, on utilise **des** compositions présentant une teneur supérieure à 60%, de préférence supérieure à 80% pour les formes G0 + G1 + G0F + G1F et **une** teneur en formes G0F + G1F est-inférieure à 50%, de préférence inférieures à 30% pour la préparation d'un médicament destiné au traitement des patients homozygotes pour la phénylalanine en position 158 du CD16 (FCGR3A-158F homozygotes) ou des patients hétérozygotes valine/phénylalanine en position 158 du CD16 (FCGR3A-158V/F).

Avantageusement, les anticorps mentionnés ci-dessus sont des IgG1.

Un autre objet de la présente description est une méthode de traitement thérapeutique comprenant l'administration d'un anticorps monoclonal chimérique, humanisé ou humain dont la structure gylcannique du domaine Fc de l'anticorps correspond à un type biantenné, avec des chaînes courtes, une faible sialylation, des mannoses et GlcNAc du point d'attache terminaux non intercalaires et une faible fucosylation à des patients homozygotes pour la phénylalanine en position 158 du CD16 (FCGR3A-158F homozygotes) ou des patients hétérozygotes valine/phénylalanine en position 158 du CD16 (FCGR3A-158V/F).

Avantageusement, les patients sont des patients homozygotes pour la phénylalanine en position 158 du CD16 (FCGR3A-158F homozygotes).
Préférentiellement, les patients traités sont en échec thérapeutique avec les anticorps actuellement disponibles ou subissent des effets secondaires indésirables.

Avantageusement, la dose de l'anticorps administrée au patient est 2 fois, 5 fois, de préférence 10 fois, 25 fois, 50 fois ou de préférence 100 fois moins élevée qu'une dose indiquée avec un anticorps de même spécificité mais de glycosylation différente ou produit dans une lignée CHO.
De manière avantageuse, la dose de l'anticorps administrée au patient est entre 2 et 5 fois, entre 5 et 10 fois, entre 5 et 25 fois, entre 5 et 50 fois ou de préférence entre 5 et 100 fois moins élevée qu'une dose d'un anticorps de même spécificité mais de glycosylation différente ou produit dans une lignée CHO.

Dans un autre aspect de l'invention, l'anticorps utilisé peut être produit dans des lignées cellulaires du type myélome de rat, par exemple YB 2/0 (ATCC n° CRL 1662). En effet, de telles cellules permettent l'obtention d'un anticorps glycosylé tel que décrit précédemment. Ainsi, dans un aspect complémentaire, la présente description porte sur l'utilisation d'un anticorps monoclonal chimérique, humanisé ou humain produit dans une lignée de myélome de rat, par exemple YB2/0 ou un de ses dérivés, pour la préparation d'un médicament destiné au traitement de patients présentant la forme FCGR3A-158V/F ou FCGR3A-158F homozygotes du CD16, en particulier des patients se trouvant en échec thérapeutique avec les anticorps actuellement disponibles ou subissant des effets secondaires indésirables justifiant l'administration de l'anticorps optimisé de l'invention. De préférence, la présente description porte sur l'utilisation d'anticorps produits dans des lignées de myélomes de rat, notamment YB2/0 et ses dérivés. Lesdits anticorps possédant la double propriété d'induire via le CD16 de forme FCGR3A-158V/F ou FCGR3A-158F homozygotes, l'ADCC mais également la production d'IFNγ ou d'autres cytokines et/ou chémokines par les cellules du système immunitaire, ceci pouvant accroître et/ou induire l'activité cytotoxique de cellules effectrices (NK, monocytes, macrophage, polynucléaires neutrophiles ...) décrites comme exprimant le récepteur CD16 sont utilisés pour la préparation d'un médicament destiné au traitement d'une population particulière de patients faibles répondeurs ou se trouvant en échec thérapeutique avec les anticorps actuellement disponibles ou subissant des effets secondaires indésirables. L'anticorps produit dans des lignées de myélome rat, notamment YB2/0 ou un de ses dérivés, présente la structure glycannique du fragment Fc telle que décrite ci-dessus, la teneur en formes G0+G1+G0F+G1F et G0F+G1F telle que décrite ci-dessus, et il induit une cytotoxicité par ADCC et la sécrétion de cytokines de la manière déjà décrite ci-dessus.

L'anticorps utilisé dans le cadre de l'invention peut être dirigé contre un antigène non ubiquitaire présent sur des cellules de donneur sain (par exemple, l'anticorps est de spécificité anti-Rhésus du globule rouge humain), ou un antigène d'une cellule pathologique ou d'un organisme pathogène pour l'homme, en particulier contre un antigène d'une cellule cancéreuse ou infectée par un virus. Il est avantageux d'utiliser les anticorps définis ci-dessus pour le traitement des cancers et des infections par des agents pathogènes pour ces patients.

Parmi les pathologies nécessitant l'administration de tels anticorps chez ces patients, on peut citer à titre d'exemple les maladies choisies parmi la maladie hémolytique du nouveau né et celles échappant à la réponse immune notamment, le Syndrome de Sezary, les leucémies myéloïdes chroniques, les leucémies lymphoïdes chroniques (LLC-B), les tumeurs solides, le cancer du sein, les pathologies liées à l'environnement visant notamment les personnes exposées aux biphényles polychlorinés, les maladies infectieuses, notamment la tuberculose, le syndrome de la fatigue chronique (CFS), les infections parasitaires comme par exemple les schistosomules ou le paludisme, en particulier chez les femmes enceintes, et les infections virales pour cibler les cellules réservoirs de virus (HIV, HCV, HBV notamment) et les cellules infectées.

Parmi les pathologies traitées on peut citer les pathologies dans lesquelles l'antigène est faiblement exprimé (antigène Rhésus D sur hématies, leucémies, leucémie lymphoïde chronique B (LLC-B), par exemple). On entend par « antigène faiblement exprimé » un nombre de sites antigéniques inférieur à 250000, de préférence inférieur à 100000 ou 50000 et de manière particulièrement avantageuse inférieur à 10000 ou 5000 par cellule cible.

Dans un aspect particulier, l'invention s'adresse à des patients présentant la forme FCGR3A-158V/F ou FCGR3A-158F homozygote du CD16 et atteints de LLC-B. Le CD20 étant très faiblement exprimé sur ces cellules tumorales, l'utilisation d'anticorps anti-CD20 selon l'invention pour traiter ces patients est particulièrement avantageuse.

Parmi les cancers, on vise plus particulièrement les cancers des cellules HLA classeII positives, les lymphomes de cellules B, les leucémies aiguës de cellules B, le lymphome de Burkitt, le lymphome de Hodgkin, les leucémies myéloïdes, les leucémies lymphoïdes chroniques (LLC-B) les lymphomes et leucémies de cellules T, les lymphomes non hodgkinien et les leucémies myéloïdes chroniques.

L'anticorps peut être un anticorps anti-HLA-DR ou un anti-CD20.

Dans un aspect particulier de l'invention, lorsque l'anticorps est un anti-CD20, l'anticorps est administré avantageusement à une dose inférieure à 187,5 mg/kg, à 75 mg/kg, à 37,5 mg/kg, 15 mg/kg, 7,5 mg/kg ou de manière préférée inférieure à 3,75 mg/kg. La dose administrée est avantageusement comprise entre 187,5 mg/kg et 75 mg/kg, ou entre 75 mg/kg et 37,5 mg/kg, entre 75 mg/kg et 15 mg/kg, entre 75 mg/kg et 7,5 mg/kg et de manière préférée entre 75 mg/kg et 3,75 mg/kg ou encore entre 15 mg/kg et 3.75 mg/kg.
De manière avantageuse, cet anticorps présente un taux ADCC supérieur à 100% et un taux de production d'IL-2 par la cellule Jurkat CD16 supérieur jusqu'à 1000 % comparé au Rituxan®.
L'anti-CD20 **utilisé dans le cadre** de l'invention peut être produit dans une lignée de myélome de rat, notamment YB2/0.

De plus, à titre d'exemple, les anticorps utilisés dans le cadre de l'invention peuvent être des anticorps de seconde génération correspondant aux anticorps disponibles actuellement listés dans le Tableau 1.

**Tableau 1**

| **Nom et marque commerciale de l'anticorps** | **Société** | **cible** | **indication** |
|---|---|---|---|
| Edrecolomab PANOREX | Centocor | anti Ep-CAM | colorectal cancer |
| Rituximab RITUXAN | Idec Licensié à Genentech/ Hoffman la roche | anti CD20 | B cell lymphoma thrombocytopenia purpura |
| Trastuzumab HERCEPTIN | Genentech Licensié à Hoffman la roche/Immunogen | anti HER2 | ovarian cancer |
| Palivizumab SYNAGIS | Medimmune Licensié à Abott | | RSV |
| Alemtuzumab CAMPATH | BTG Licensié à Schering | anti CD52 | leukemia |
| ibritumomab tiuxetan ZEVALIN | IDEC Licensié à Schering | anti CD20 | NHL |
| Cetuximab IMC-C225 | Merck /BMS / Imclone | anti HER1 | cancers |
| | | | |
| Bevacizumab *AVASTIN* | Genentech/ Hoffman la roche | anti VEGF | Poumon, colorectal, rénal cancers |
| Epratuzumab | Immumedics/ Amgen | anti CD22 | cancers: non hogkin lymphoma |
| Hu M195Mab | Protein Design Labs | ND | cancers |
| MDX-210 | Medarex/Immuno-Designed Molécules | Bi spécifique HER2Neu/C D64 | Breast, ovarian, prostate cancers |
| BEC2 Mitumomab | Imclone | anti GD3 | Small cell lung carcinoma |
| Oregovomab *OVAREX* | Altarex | anti CA125 | Ovarian cancer |
| Ecromeximab KW-2971 | Kyowa-Hakko | anti GD | malignant melanoma |
| ABX-EGF | Abgenix | EGF | cancers |
| MDX010 | Medarex | ND | Cancers |
| XTL 002 | XTL biopharmaceuticals | ND | anti-viral : HCV |
| H11 SCFV | viventia biotech | ND | cancers |
| 4B5 | viventia biotech | anti GD2 | Cancers |
| XTL 001 | XTL biopharmaceuticals | ND | anti-viral : HBV |
| MDX-070 | MEDAREX | Anti-PSMA | Prostate cancer |
| TNX-901 | TANOX | anti CD-23 | |
| IDEC-114 | IDEC | inhibition ProteinC | non-Hodgkin lymphoma |

D'autres anticorps peuvent être sélectionnés parmi les anti Ep-CAM, anti HER2, anti CD52, anti HER1, anti GD3, anti CA125, anti GD, anti GD2, anti CD-23 et anti ProteinC ; anti-KIR3DL2, anti-EGFR, anti-CD25, anti-CD38, anti-CD30, anti-CD33, anti-CD44, des anti-idiotypes spécifiques d'inhibiteurs par exemple de facteurs de coagulation, les anti-viraux : HIV, HBV, HCV et RSV.

Dans un aspect préféré, l'anticorps est un anti-HLA-DR. Cet anticorps présente un taux ADCC supérieure de 100 % et un taux de production d'IL2 par la cellule Jurkat CD16, ou d'IFNγ par une cellule effectrice du système immunitaire exprimant le récepteur CD16 supérieur jusqu'à 1000 comparé au même anticorps exprimé dans la lignée CHO, lignée d'expression du Remitogen®.
L'anti-HLA-DR peut être produit dans une lignée de myélome de rat, notamment YB2/0.

La présente description porte également sur l'utilisation d'un anticorps décrit ci-dessus pour la fabrication d'un médicament destiné à induire l'expression d' IL-1, IL4, IL12, IL18, IL21, IL-2, IL-3, IL-5, IL-6, IL-7, IL-8, IL-9, IL-10, IFNα, , IFNβ, TNFα, TGFβ, IP10 et IFNγ par les cellules effectrices naturelles du système immunitaire, ledit médicament étant utile notamment pour le traitement du cancer et des infections chez les patients se trouvant en échec thérapeutique avec les anticorps actuellement disponibles ou subissant des effets secondaires indésirables justifiant l'administration de l'anticorps optimisé de l'invention et notamment présentant la forme V/F158 ou F/F158 du CD16.

D'autres aspects et avantages de l'invention seront décrits dans les exemples qui suivent, qui doivent être considérés comme illustratifs.

### Légendes de figures

Figure 1 : Activité ADCC de l'anticorps monoclonal anti-Rhésus D R297 (IgG1 T125 produit dans YB2/0) et des anticorps polyclonaux WinRho en présence de cellules effectrices (PBMC : Peripheral Blood Mononuclear Cells) de différents donneurs de sang et d'immunoglobulines polyvalentes (Tégéline 500µg/ml).
Figure 2: ADCC induit par les anticorps anti-Rhésus D (anticorps WinRho polyclonaux, T125 produits dans YB2/0 et T125 produits dans CHO) sur des cellules NK de donneurs de phénotype CD16 FCGR3A-158F homozygotes.
Figure 3 : ADCC induit par les anticorps anti-Rhésus D (anticorps WinRho polyclonaux, T125 produits dans YB2/0 et T125 produits dans CHO) sur des cellules NK de donneurs de phénotype CD16 FCGR3A-158V homozygotes.
Figure 4 : Activation de Jurkat CD16 FCGR3A-158F homozygotes par les anticorps anti-Rhésus D T125 exprimés dans YB2/0 et CHO.
Figure 5 : Activation de Jurkat CD16 FCGR3A-158V homozygotes par les anticorps anti-Rhésus D T125 exprimés dans YB2/0 et CHO.
Figure 6 : Activation de Jurkat CD16 FCGR3A-158F homozygotes induite par les anticorps anti-HLA-DR exprimés dans YB2/0 et CHO.
Figure 7 : Activation de Jurkat CD16 FCGR3A-158V homozygotes induite par les anticorps anti-HLA-DR exprimés dans YB2/0 et CHO.

### Exemples

### Exemple 1 : Activité ADCC de l'anticorps monoclonal anti-Rhésus D R297 comparée aux anticorps polyclonaux anti-D sur un groupe de 107 donneurs de sang.

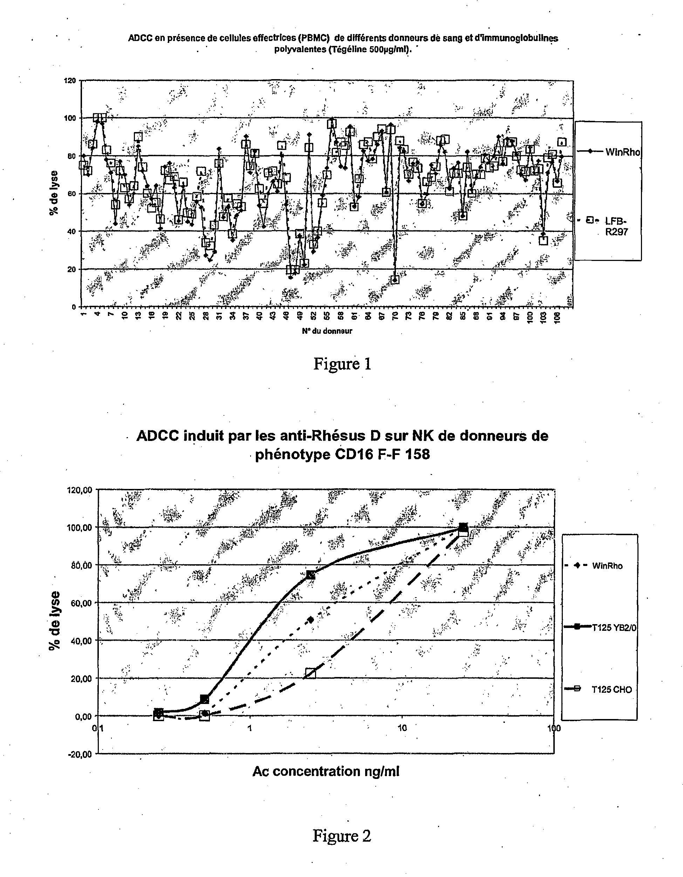

Les capacités respectives de l'anticorps monoclonal anti-Rhésus D R297 et des anticorps polyclonaux anti-D à lyser des hématies en présence de cellules effectrices de différents donneurs individuels sont comparées (figure 1).

Les cellules effectrices proviennent d'un groupe de107 donneurs de sang. Les cellules mononucléées (PBMC) sont isolées à partir d'une poche de sang par centrifugation sur un gradient de Ficoll (Pack Plus Pharmacia). Les plaquettes sont éliminées par centrifugation (190g, 15min) et les globules rouges résiduels sont lysés au NH4Cl. Les cellules sont lavées et resuspendues à 8x10⁷cells/ml en IMDM. Les globules rouges obtenus à partir de concentrés thérapeutiques (group O, Rhésus +) sont traités pendant 10 min à la papaïne (1mg/ml) puis lavés trois fois en tampon salin et ajustés à la concentration de 4x10⁷/ml ou 2x10⁷/ml (test NK) en IMDM.

Le test s'effectue en plaque de 96 puits (NUNC). Les surnageants de culture ou les anticorps purifiés (100µl à 200ng/ml in IMDM + 0.5% SVF), les cellules effectrices (25 µl), les globules rouges (25µl) et les immunoglobulines polyvalentes (Tégéline, LFB) (50µl) sont incubés pendant 16h à 37°C sous atmosphère enrichi en CO2 %. Pour la lyse non spécifique, les cellules effectrices sont remplacées par de l'IMDM. Après 16h à 37°C, les plaques sont centrifugées. 60µl du surnageants sont prélevés et mélangés avec 60µl de 2.7diaminofluorenee (DAF, Sigma).
Après 5 min, la DO est mesurée à 620nm
Le pourcentage de lyse est estimé en utilisant une gamme de calibration obtenues avec différentes dilutions de globules rouges lysés au NH4Cl, correspondant à 100, 75, 50, 25 et 0% de lyse respectivement.

Sur la base d'étude génotypique menées sur des donneurs de même origine géographique, on estime dans la présente étude que la répartition moyenne des 107 donneurs en ce qui concerne le polymorphisme du CD16 est de 27 FCGR3A-158F homozygotes, 20 FCGR3A-158V homozygotes et 60 FCGR3A-158V/F.

Les résultats montrent une grande variabilité dans la capacité des cellules effectrices des différents sujets à induire une lyse des hématies Rhésus positif quels que soient les anticorps testés. Les anticorps exprimés dans la lignée cellulaire YB2/0 présentent une activité cytolytique comparable à celle des anticorps polyclonaux, quel que soit le donneur étudié, et par conséquent quel que soit le polymorphisme du CD16 des cellules effectrices du donneur (voir figure 1).

### Exemple 2 : Efficacité ADCC des anticorps produits dans CHO et YB2/0 en fonction du polymorphisme du CD16.

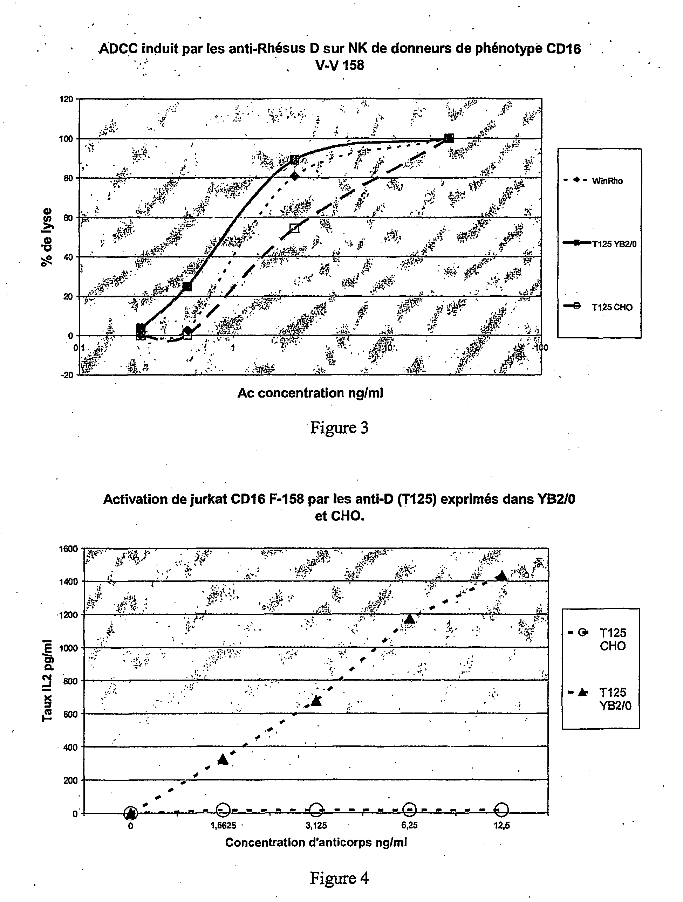

La même séquence codant pour une IgG1 spécifique de l'antigène Rhésus D a été transfectée dans les lignées cellulaires CHO et YB2/0. Les anticorps ont été incubés avec des hématies Rhésus positif (cellules cibles) et des cellules NK provenant de 6 donneurs différents (3 FCGR3A-158V homozygotes et 3 FCGR3A-158F homozygotes) préalablement génotypés pour leur phénotype CD16 en position 158. Les cellules NK sont isolées en utilisant la technique de séparation sur billes magnétiques (MACS) de chez Myltenyi. Les cellules NK sont lavées et resuspendues à 2x10⁷/ml et/ou 6x10⁷/ml en IMDM. Les globules rouges sont ajustés à la concentration de 2x10⁷/ml en IMDM. La Tégéline est remplacée par de l'IMDM. En dehors de ces modifications, le test est identique au test ADCC avec PBMC.
L'activité cytotoxique des anticorps sur les hématies (ADCC) a été évaluée (figure 2 et figure 3).
L'anticorps produit dans la lignée CHO induit une lyse plus faible que l'anticorps produit dans YB2/0 quel que soit le phénotype du donneur. L'anticorps R297 exprimé dans la lignée YB2/0 induit dès les plus faibles concentrations une plus forte activité cytolytique. A la concentrations maximale de 25ng/ml, les deux anticorps induisent le même pourcentage d'ADCC.

Aux concentrations inférieures à 25ng/ml, la différence de lyse entre l'anticorps produit dans CHO et celui produit dans YB2/0 est plus importante chez les donneurs FCGR3A-158F homozygotes que les donneurs FCGR3A-158V homozygotes. A la concentration de 2.5ng/ml, en présence des cellules NK FCGR3A-158V homozygotes, l'anticorps produit par CHO induit 54% de lyse alors que celui produit par YB2/0 induit 89% de lyse soit 56% d'augmentation. Par contre, à la même concentration, en présence des cellules NK FCGR3A-158F homozygotes, l'anticorps produit par CHO n'induit que 22% de lyse alors que celui produit par YB2/0 induit 74% de lyse soit 236% d'augmentation.
L'anticorps exprimé dans la lignée YB2/0 s'avère donc être un bien meilleur produit pour traiter les patients donnant une lyse faible avec les anticorps produits dans CHO. Ainsi, la différence d'activité ADCC entre les patients FCGR3A-158V homozygotes et FCGR3A-158F homozygotes est plus faible avec l'anticorps exprimé dans YB2/0 (89 et 74%) par rapport à celle observée avec l'anticorps exprimé dans CHO (56 et 22%). Les anticorps optimisés présentent une réponse qui apparaît donc moins dépendante des formes polymorphiques du CD16.
D'autre part, l'anticorps monoclonal exprimé dans YB2/0 induit toujours une lyse supérieure ou égale aux anticorps polyclonaux.

### Exemple 3 : Comparaison de l'activation de cellules Jurkat CD16 FCGR3A-158F homozygotes et Jurkat CD16 FCGR3A-158F homozygotes induite par les anticorps anti-Rhésus produits dans CHO et YB2/0 respectivement : évaluation de la production d'IL2.

Ce test estime la capacité des anticorps à se fixer sur le récepteur CD16 (Fc gamma RIII) exprimé sur les cellules Jurkat CD16 et à induire la sécrétion d'IL2.
La même séquence codant pour une IgG1 (T125) spécifique de l'antigène Rhésus D a été transfectée dans les lignées cellulaires CHO et YB2/0. Les anticorps sont incubés avec des hématies Rhésus positif (cellule cible) et des cellules Jurkat CD16 (cellules effectrices). Deux types de cellules Jurkat ont été utilisées : 1- des cellules transfectées avec le gène codant pour un RFc portant l'acide aminé phénylalanine F en position 158 (forme F), 2- des cellules transfectées avec le gène codant pour un RFc portant l'acide aminé valine V en position 158 (forme V). La quantité de cytokine (IL2) sécrétée par les cellules Jurkat CD16 a été mesurée par ELISA.
En plaque de 96 puits in mélange :
Anticorps : 50µl d'une dilution de 50; 37,5; 25; 18,75 ; 12,5 ; 9,4 ;6,25 ; 3,125 ng/ml en IMDM (Iscove's Modified Dulbecco's) Medium 5% SVF (sérum de veau foetal)
   - PMA 50µl d'une dilution à 40ng/ml en IMDM 5% SVF
   - Hématies traitées à la papaïne. 50µl à 8x10⁶/ml en IMDM 5% SVF
   - Jurkat CD16. 50µl à 2x10⁶/ml en IMDM 5% SVF
Incubation 1 nuit à 37°C
Puis centrifugation des plaques, prélèvement de 100µl de surnageants et dosage d'IL2 avec le kit commercial (Quantikine de chez R/D). Lecture à 450nm.

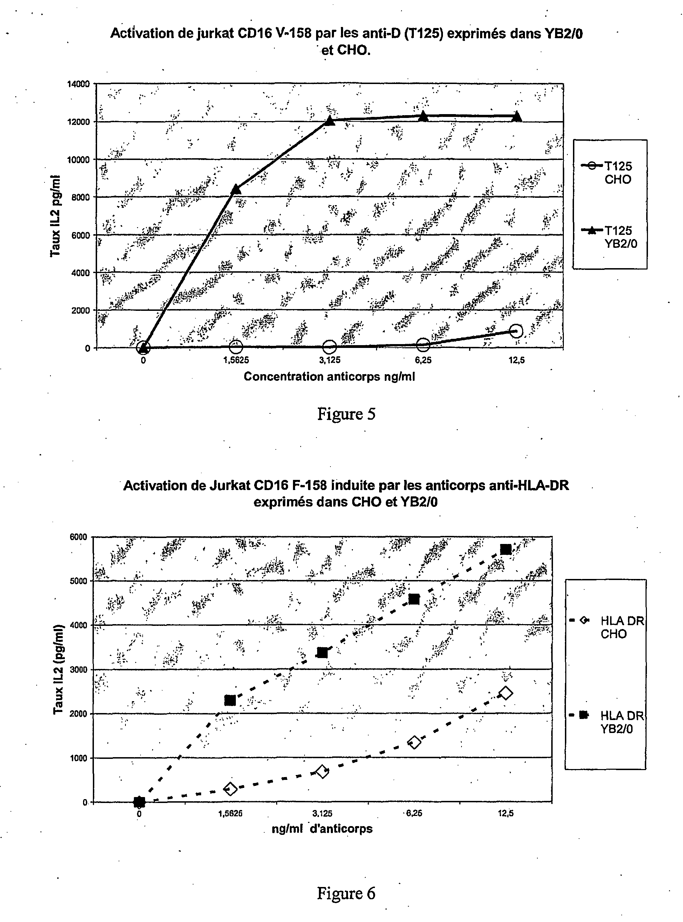

L'anticorps exprimé dans la lignée YB2/0 est capable d'induire une plus forte sécrétion d'IL2 contrairement à l'anticorps exprimé dans CHO, et cela quel que soit le phénotype CD16 (figure 4 et figure 5).

L'anticorps produit dans CHO n'induit pas de sécrétion d'IL2 à partir de Jurkat CD16 FCGR3A-158F homozygotes et seulement une très faible production d'IL2 avec la forme FCGR3A-158V homozygotes.

Une des particularités du système Rhésus étant la faible expression de l'antigène à la surface membranaire, il apparaît que dans ces conditions, l'anticorps exprimé dans la lignée YB2/0 s'avère être un bien meilleur produit pour activer les cellules effectrices porteuses de la forme FCGR3A-158F homozygotes du CD16 qui n'apparaissent pas activables avec l'anticorps produit dans CHO. En ce qui concenre la forme FCGR3A-158V homozygotes, de très faibles quantités d'anticorps produits par YB2/0 (<1,56) permettent d'induire une activation comparable à celle obtenue avec de plus fortes concentrations d'anticorps produits dans CHO (12,5ng/ml).

Avec la forme FCGR3A-158F homozygotes et à la concentration de 12,5ng/ml, l'anticorps produit dans CHO induit une sécrétion d'IL2 (18 pg/ml) inférieure à 2% de celle induite par l'anticorps produit dans YB2/0 (1435 pg/ml). Ceci correspond à un gain de plus de 7000% lorsque l'on utilise l'anticorps produit dans YB2/0 par rapport à l'anticorps produit dans CHO.

Avec la forme FCGR3A-158V homozygotes et à la concentration de 12,5ng/ml, l'anticorps produit dans CHO induit une sécrétion d'IL2 (869 pg/ml) inférieure à 8% de celle induite par l'anticorps produit dans YB2/0 (12312 pg/ml). Ceci correspond à un gain de plus de 1300% lorsque l'on utilise l'anticorps produit dans YB2/0 par rapport à l'anticorps produit dans CHO.

### Exemple 4 : Comparaison de l'activation des cellules Jurkat CD16 FCGR3A-158F homozygotes et Jurkat CD16 FCGR3A-158V homozygotes induite par deux anticorps anti-HLA-DR exprimés dans CHO et YB2/0 respectivement : évaluation de la sécrétion d'IL2 .

La même séquence codant pour une IgG1 spécifique de l'antigène HLA-DR a été transfectée dans les lignées cellulaires CHO et YB2/0. Les anticorps sont incubés avec des cellules Raji (cellule cible HLA-DR positives) et des cellules Jurkat CD16 (cellules effectrices). Deux types de cellules Jurkat ont été utilisées : 1- des cellules transfectées avec le gène codant pour un R Fc portant l'acide aminé phénylalanine F en position 158 (forme F), 2- des cellules transfectées avec le gène codant pour un RFc ayant l'acide aminé valine V en position 158 (forme V). La quantité de cytokines (IL2) sécrétée par les cellules Jurkat CD16 a été mesurée par ELISA.

En plaque de 96 puits in mélange :
Anticorps : 50µl d'une dilution de 50; 37,5; 25; 18,75 ; 12,5 ; 9,4 ;6,25 ; 3,125 ng/ml en IMDM 5% SVF
   - PMA 50µl d'une dilution à 40ng/ml en IMDM 5% SVF
   - Cellules Raji : 50µl à 6x10⁵/ml en IMDM 5% SVF
   - Jurkat CD16. 50µl à 20x10⁶/ml en IMDM 5% SVF
Incubation 1 nuit à 37°C
Puis centrifugation des plaques, prélèvement de 100µl de surnageants et dosage d'IL2 avec le kit commercial (Quantikine de chez R/D). Lecture à 450nm.

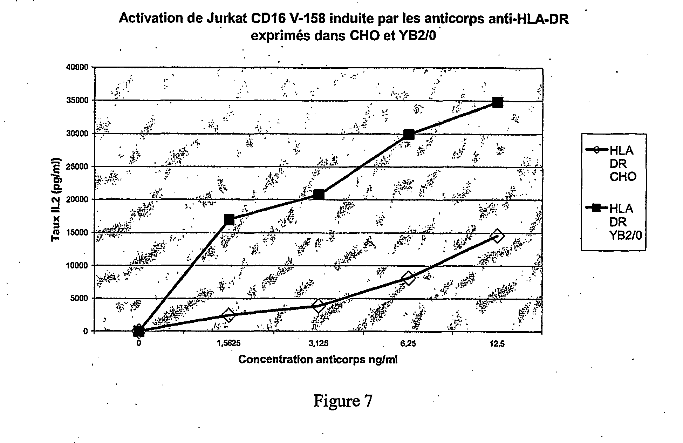

L'anticorps exprimé dans la lignée YB2/0 est capable d'induire une plus forte sécrétion d'IL2 contrairement à l'anticorps exprimé dans CHO, et cela quel que soit le phénotype CD16 (figure 6 et figure 7).

Contrairement au système Rhésus sur les hématies, l'expression de l'antigène HLA-DR à la surface membranaire de la cellule Raji n'est pas faible (200 000 à 400 000 copies). Dans ces conditions, il apparaît que l'anticorps exprimé dans la lignée YB2/0 s'avère être un bien meilleur produit pour activer les cellules effectrices porteuses de la forme F158 ainsi que V158 du CD16 et cela plus particulièrement aux faibles concentrations d'anticorps. Ainsi à la concentration de 1,56 ng/ml, l'anticorps produit dans CHO induit une sécrétion d'IL2 (2410 pg/ml) inférieure à 15% de celle induite par l'anticorps produit dans YB2/0 (16952 pg/ml). Ceci correspond à un gain de plus de 600% lorsque l'on utilise l'anticorps produit dans YB2/0 par rapport à l'anticorps produit dans CHO.

A la concentration de 12,5 ng/ml, l'anticorps produit dans CHO induit une sécrétion d'IL2 (14597 pg/ml) inférieure à 45% de celle induite par l'anticorps produit dans YB2/0 (34823 pg/ml). Ceci correspond à un gain de plus de 100% lorsque l'on utilise l'anticorps produit dans YB2/0 par rapport à l'anticorps produit dans CHO.

L'anticorps exprimé dans la lignée YB2/0 s'avère donc être un bien meilleur produit pour induire la sécrétion de cytokines de cellules effectrices porteuses de la forme polymorphique V158 (FCGR3A-158V homozygotes) et F158 (FCGR3A-158F homozygotes).

## Revendications

1. Utilisation d'une composition d'anticorps monoclonal chimérique, humanisé ou humain, ladite composition présentant une teneur supérieure à 60% pour les formes G0 + G1 + G0F + G1F et une teneur pour les formes G0F + G1F inférieure à 50%, pour la préparation d'un médicament destiné au traitement des patients homozygotes pour la phénylalanine en position 158 du CD16 (FCGR3A-158F homozygotes) ou des patients hétérozygotes valine/phénylalanine en position 158 du CD16 (FCGR3A-158V/F).

2. Utilisation d'une composition d'anticorps monoclonal chimérique, humanisé ou humain anti-Rhésus du globule rouge humain, ladite composition présentant une teneur supérieure à 60% pour les formes G0 + G1 + G0F + G1F et une teneur pour les formes G0F + G1F inférieure à 50%, pour la préparation d'un médicament destiné au traitement de la maladie hémolytique du nouveau-né chez des patients homozygotes pour la phénylalanine en position 158 du CD16 (FCGR3A-158F homozygotes) ou des patients hétérozygotes valine/phénylalanine en position 158 du CD16 (FCGR3A-158V/F).

3. Utilisation d'une composition d'anticorps monoclonal chimérique, humanisé ou humain dirigé contre un antigène d'une cellule cancéreuse, ladite composition présentant une teneur supérieure à 60% pour les formes G0 + G1 + G0F + G1F et une teneur pour les formes G0F + G1F inférieure à 50%, pour la préparation d'un médicament destiné au traitement d'un cancer chez des patients homozygotes pour la phénylalanine en position 158 du CD16 (FCGR3A-158F homozygotes) ou des patients hétérozygotes valine/phénylalanine en position 158 du CD16 (FCGR3A-158V/F).

4. Utilisation selon la revendication 3, **caractérisée en ce que** l'anticorps est un anti-HLA-DR ou un anti-CD20.

5. Utilisation selon la revendication 4, **caractérisée en ce que** le médicament est destiné au traitement des cancers des cellules HLA classe II positives, des lymphomes de cellules B, des leucémies aiguës de cellules B, des lymphome de Burkitt, du lymphome de Hodgkin, des leucémies myéloïdes, des leucémies lymphoïdes chroniques B (LLC-B), des lymphomes et leucémies de cellules T, des lymphomes non hodgkinien et des leucémies myéloïdes chroniques.

6. Utilisation selon la revendication 3, **caractérisée en ce que** l'anticorps est un anticorps choisi parmi les anti Ep-CAM, anti HER2, anti CD52, anti HER1, anti GD3, anti CA125, anti GD, anti GD2, anti CD-23 et anti ProteinC, anti-KIR3DL2, anti-EGFR, anti-CD25, anti-CD38, anti-CD30, anti-CD33, et anti-CD44.

7. Utilisation selon l'une quelconque des revendications 1 à 6, **caractérisée en ce que** ladite composition présente une teneur supérieure à 80% pour les formes G0 + G1 + G0F + G1F.

8. Utilisation selon l'une quelconque des revendications 1 à 7, **caractérisée en ce que** les formes G0F + G1F sont inférieures à 30%.

9. Utilisation selon l'une quelconque des revendications 1 à 8, **caractérisée en ce que** les anticorps sont des IgG1.

10. Utilisation selon l'une quelconque des revendications 1 à 9, **caractérisée en ce que** les patients sont des patients homozygotes pour la phénylalanine en position 158 du CD16 (FCGR3A-158F homozygotes).

11. Composition d'anticorps monoclonal chimérique, humanisé ou humain, ladite composition présentant une teneur supérieure à 60% pour les formes G0 + G1 + G0F + G1F et une teneur pour les formes G0F + G1F inférieure à 50%, pour son utilisation dans le traitement des patients homozygotes pour la phénylalanine en position 158 du CD16 (FCGR3A-158F homozygotes) ou des patients hétérozygotes valine/phénylalanine en position 158 du CD16 (FCGR3A-158V/F).

12. Composition d'anticorps monoclonal chimérique, humanisé ou humain anti- Rhésus du globule rouge humain, ladite composition présentant une teneur supérieure à 60% pour les formes G0 + G1 + G0F + G1F et une teneur pour les formes G0F + G1F inférieure à 50%, pour son utilisation dans le traitement de la maladie hémolytique du nouveau-né chez des patients homozygotes pour la phénylalanine en position 158 du CD16 (FCGR3A-158F homozygotes) ou des patients hétérozygotes valine/phénylalanine en position 158 du CD16 (FCGR3A-158V/F).

13. Composition d'anticorps monoclonal chimérique, humanisé ou humain dirigé contre un antigène d'une cellule cancéreuse, ladite composition présentant une teneur supérieure à 60% pour les formes G0 + G1 + G0F + G1F et une teneur pour les formes G0F + G1F inférieure à 50%, pour son utilisation dans le traitement d'un cancer chez des patients homozygotes pour la phénylalanine en position 158 du CD16 (FCGR3A-158F homozygotes) ou des patients hétérozygotes valine/phénylalanine en position 158 du CD16 (FCGR3A-158V/F).

14. Composition selon la revendication 13 pour son utilisation selon la revendication 13, **caractérisée en ce que** l'anticorps est un anti-HLA-DR ou un anti-CD20.

15. Composition selon la revendication 14 pour son utilisation selon la revendication 14, **caractérisée en ce que** le médicament est destiné au traitement des cancers des cellules HLA classe II positives, des lymphomes de cellules B, des leucémies aiguës de cellules B, des lymphome de Burkitt, du lymphome de Hodgkin, des leucémies myéloïdes, des leucémies lymphoïdes chroniques B (LLC-B), des lymphomes et leucémies de cellules T, des lymphomes non hodgkinien et des leucémies myéloïdes chroniques.

16. Composition selon la revendication 13 pour son utilisation selon la revendication 13, **caractérisée en ce que** l'anticorps est un anticorps choisi parmi les anti Ep-CAM, anti HER2, anti CD52, anti HER1, anti GD3, anti CA125, anti GD, anti GD2, anti CD-23 et anti ProteinC, anti-KIR3DL2, anti-EGFR, anti-CD25, anti-CD38, anti-CD30, anti-CD33, et anti-CD44.

17. Composition selon l'une quelconque des revendications 11 à 16 pour son utilisation selon l'une quelconque des revendications 11 à 16, **caractérisée en ce que** ladite composition présente une teneur supérieure à 80% pour les formes G0 + G1 + G0F + G1F.

18. Composition selon l'une quelconque des revendications 11 à 17 pour son utilisation selon l'une quelconque des revendications 11 à 17, **caractérisée en ce que** les formes G0F + G1F sont inférieures à 30%.

19. Composition selon l'une quelconque des revendications 11 à 18 pour son utilisation selon l'une quelconque des revendications 11 à 18, **caractérisée en ce que** les anticorps sont des IgG1.

20. Composition selon l'une quelconque des revendications 11 à 19 pour son utilisation selon l'une quelconque des revendications 11 à 19, **caractérisée en ce que** les patients sont des patients homozygotes pour la phénylalanine en position 158 du CD16 (FCGR3A-158F homozygotes).

## Patentansprüche

1. Verwendung einer Zusammensetzung aus chimären, humanen, oder humanisierten monoklonalen Antikörpern, wobei die Zusammensetzung einen Gehalt an den Formen G0 + G1 + G0F + G1F von über 60 % und einen Gehalt an den Formen G0F + G1F von unter 50 % aufweist, zur Herstellung eines Medikaments zur Behandlung von Patienten, die für das Phenylalanin an Position 158 von CD 16 homozygot sind (FCGR3A-158F-homozygot), oder von Patienten, die an Position 158 von CD16 Valin/Phenylalanin-heterozygot sind (FCGR3A-158V/F).

2. Verwendung einer Zusammensetzung aus chimären, humanen, oder humanisierten monoklonalen Antikörpern gegen den Rhesusfaktor von humanen Erythrozyten, wobei die Zusammensetzung einen Gehalt an den Formen G0 + G1 + G0F + G1F von über 60 % und einen Gehalt an den Formen G0F + G1F von unter 50 % aufweist, zur Herstellung eines Medikaments zur Behandlung der hämolytischen Krankheit von Neugeborenen bei Patienten, die für das Phenylalanin an Position 158 von CD 16 homozygot sind (FCGR3A-158F-homozygot), oder von Patienten, die an Position 158 von CD16 Valin/Phenylalanin-heterozygot sind (FCGR3A-158V/F).

3. Verwendung einer Zusammensetzung aus chimären, humanen, oder humanisierten monoklonalen Antikörpern, die gegen ein Antigen einer Krebszelle gerichtet sind, wobei die Zusammensetzung einen Gehalt an den Formen G0 + G1 + G0F + G1F von über 60 % und einen Gehalt an den Formen G0F + G1F von unter 50 % aufweist, zur Herstellung eines Medikaments zur Behandlung von Krebs bei Patienten, die für das Phenylalanin an Position 158 von CD 16 homozygot sind (FCGR3A-158F-homozygot), oder von Patienten, die an Position 158 von CD16 Valin/Phenylalanin-heterozygot sind (FCGR3A-158V/F).

4. Verwendung gemäß Anspruch 3, **dadurch gekennzeichnet, dass** der Antikörper ein Anti-HLA-DR oder ein Anti-CD20 ist.

5. Verwendung gemäß Anspruch 4, **dadurch gekennzeichnet, dass** das Medikament zur Behandlung von Krebs HLA-Klasse-II-positiver Zellen, B-Zell-Lymphomen, akuten B-Zell-Leukämien, Burkitt-Lymphom, Hodgkin-Lymphom, myeloischen Leukämien, chronischen lymphatischen Leukämien vom B-Zelltyp (B-CLL), T-Zell-Lymphomen und -Leukämien, Non-Hodgkin-Lymphomen und chronischen myeloischen Leukämien bestimmt ist.

6. Verwendung gemäß Anspruch 3, **dadurch gekennzeichnet, dass** der Antikörper ein Antikörper ist, der aus Anti-Ep-CAM, Anti-HER2, Anti-CD52, Anti-HER1, Anti-GD3, Anti-CA125, Anti-GD, Anti-GD2, Anti-CD-23 und Anti-Protein C, Anti-KIR3DL2, Anti-EGFR, Anti-CD25, Anti-CD38, Anti-CD30, Anti-CD33 und Anti-CD44 ausgewählt ist.

7. Verwendung gemäß irgendeinem der Ansprüche 1 bis 6, **dadurch gekennzeichnet, dass** die Zusammensetzung einen Gehalt an den Formen G0 + G1 + G0F + G1F von über 80 % aufweist.

8. Verwendung gemäß irgendeinem der Ansprüche 1 bis 7, **dadurch gekennzeichnet, dass** die Formen G0F + G1F mit weniger als 30 % vorliegen.

9. Verwendung gemäß irgendeinem der Ansprüche 1 bis 8, **dadurch gekennzeichnet, dass** die Antikörper IgG1 sind.

10. Verwendung gemäß irgendeinem der Ansprüche 1 bis 9, **dadurch gekennzeichnet, dass** die Patienten homozygot für das Phenylalanin an Position 158 von CD16 sind (FCGR3A-158F-homozygot).

11. Zusammensetzung aus chimären, humanen, oder humanisierten monoklonalen Antikörpern, wobei die Zusammensetzung einen Gehalt an den Formen G0 + G1 + G0F + G1F von über 60 % und einen Gehalt an den Formen G0F + G1F von unter 50 % aufweist, zur Verwendung bei der Behandlung von Patienten, die für das Phenylalanin an Position 158 von CD 16 homozygot sind (FCGR3A-158F-homozygot), oder von Patienten, die an Position 158 von CD16 Valin/Phenylalaninheterozygot sind (FCGR3A-158V/F).

12. Zusammensetzung aus chimären, humanen, oder humanisierten monoklonalen Antikörpern gegen den Rhesusfaktor von humanen Erythrozyten, wobei die Zusammensetzung einen Gehalt an den Formen G0 + G1 + G0F + G1F von über 60 % und einen Gehalt an den Formen G0F + G1F von unter 50 % aufweist, zur Verwendung bei der Behandlung der hämolytischen Krankheit von Neugeborenen bei Patienten, die für das Phenylalanin an Position 158 von CD 16 homozygot sind (FCGR3A-158F-homozygot), oder von Patienten, die an Position 158 von CD16 Valin/Phenylalanin-heterozygot sind (FCGR3A-158V/F).

13. Zusammensetzung aus chimären, humanen, oder humanisierten, monoklonalen Antikörpern, die gegen ein Antigen einer Krebszelle gerichtet sind, wobei die Zusammensetzung einen Gehalt an den Formen G0 + G1 + G0F + G1F von über 60 % und einen Gehalt an den Formen G0F + G1F von unter 50 % aufweist, zur Verwendung bei der Behandlung von Krebs bei Patienten, die für das Phenylalanin an Position 158 von CD 16 homozygot sind (FCGR3A-158F-homozygot), oder von Patienten, die an Position 158 von CD16 Valin/Phenylalaninheterozygot sind (FCGR3A-158V/F).

14. Zusammensetzung gemäß Anspruch 13, zur Verwendung gemäß Anspruch 13, **dadurch gekennzeichnet, dass** der Antikörper ein Anti-HLA-DR oder ein Anti-CD20 ist.

15. Zusammensetzung gemäß Anspruch 14 zur Verwendung gemäß Anspruch 14, **dadurch gekennzeichnet, dass** das Medikament zur Behandlung von Krebs HLA-Klasse-II-positiver Zellen, B-Zell-Lymphomen, akuten B-Zell-Leukämien, Burkitt-Lymphom, Hodgkin-Lymphom, myeloischen Leukämien, chronischen lymphatischen Leukämien vom B-Zelltyp (B-CLL), T-Zell-Lymphomen und -Leukämien, Non-Hodgkin-Lymphomen und chronischen myeloischen Leukämien bestimmt ist.

16. Zusammensetzung gemäß Anspruch 13 zur Verwendung gemäß Anspruch 13, **dadurch gekennzeichnet, dass** der Antikörper ein Antikörper ist, der aus Anti-Ep-CAM, Anti-HER2, Anti-CD52, Anti-HER1, Anti-GD3, Anti-CA125, Anti-GD, Anti-GD2, Anti-CD23 und Anti-Protein C, Anti-KIR3DL2, Anti-EGFR, Anti-CD25, Anti-CD38, Anti-CD30, Anti-CD33 und Anti-CD44 ausgewählt ist.

17. Zusammensetzung gemäß irgendeinem der Ansprüche 11 bis 16 zur Verwendung gemäß irgendeinem der Ansprüche 11 bis 16, **dadurch gekennzeichnet, dass** die Zusammensetzung einen Gehalt an den Formen G0 + G1 + G0F + G1F von über 80 % aufweist.

18. Zusammensetzung gemäß irgendeinem der Ansprüche 11 bis 17 zur Verwendung gemäß irgendeinem der Ansprüche 11 bis 17, **dadurch gekennzeichnet, dass** die Formen G0F + G1F mit weniger als 30 % vorliegen.

19. Zusammensetzung gemäß irgendeinem der Ansprüche 11 bis 18 zur Verwendung gemäß irgendeinem der Ansprüche 11 bis 18, **dadurch gekennzeichnet, dass** die Antikörper IgG1 sind.

20. Zusammensetzung gemäß irgendeinem der Ansprüche 11 bis 19 zur Verwendung gemäß irgendeinem der Ansprüche 11 bis 19, **dadurch gekennzeichnet, dass** die Patienten homozygot für das Phenylalanin an Position 158 von CD16 sind (FCGR3A-158F-homozygot).

## Claims

1. Use of a chimeric, humanised, or human monoclonal antibody composition, said composition having a concentration of over 60% for the forms G0 + G1 + G0F + G1F and a concentration for the forms G0F + G1F lower than 50%, for the preparation of a drug intended for the treatment of patients homozygous for phenylalanine in position 158 of CD16 (FCGR3A-158F homozygotes) or patients heterozygous for valine/phenylalanine in position 158 of CD16 (FCGR3A-158V/F).

2. Use of a chimeric, humanised, or human monoclonal anti-Rhesus of human red blood cell antibody composition, said composition having a concentration of over 60% for the forms G0 + G1 + G0F + G1F and a concentration for the forms G0F + G1F lower than 50%, for the preparation of a drug intended for the treatment of haemolytic disease of the newborn in patients homozygous for phenylalanine in position 158 of CD16 (FCGR3A-158F homozygotes) or patients heterozygous for valine/phenylalanine in position 158 of CD16 (FCGR3A-158V/F).

3. Use of a chimeric, humanised, or human monoclonal antibody composition targeted against a cancer cell antigen, said composition having a concentration of over 60% for the forms G0 + G1 + G0F + G1F and a concentration for the forms G0F + G1F lower than 50%, for the preparation of a drug intended for the treatment of cancer in patients homozygous for phenylalanine in position 158 of CD16 (FCGR3A-158F homozygotes) or patients heterozygous for valine/ phenylalanine in position 158 of CD16 (FCGR3A-158V/F).

4. Use according to claim 3, **characterised in that** the antibody is an anti-HLA-DR or an anti-CD20.

5. Use according to claim 4, **characterised in that** the drug is intended for the treatment of cancers of HLA class-II positive cells, B-cell lymphomas, acute B-cell leukaemias, Burkitt's lymphomas, Hodgkin's lymphoma, myeloid leukaemias, B-cell chronic lymphoid leukaemias (B-CLL), T-cell leukaemias and lymphomas, non-Hodgkin's lymphomas, and chronic myeloid leukaemias.

6. Use according to claim 3, **characterised in that** the antibody is an antibody selected from anti Ep-CAM, anti HER2, anti CD52, anti HER1, anti GD3, anti CA125, anti GD, anti GD2, anti CD-23 and anti Protein C, anti-KIR3DL2, anti-EGFR, anti-CD25, anti-CD38, anti-CD30, anti-CD33 and anti-CD44.

7. Use according to any of claims 1 to 6, characterised that said composition has a concentration of over 80% for the forms G0 + G1 + G0F + G1F.

8. Use according to any of claims 1 to 7, **characterised in that** the forms G0F + G1F are lower than 30%.

9. Use according to any of claims 1 to 8, **characterised in that** the antibodies are IgG1.

10. Use according to any of claims 1 to 9, **characterised in that** the patients are homozygous for phenylalanine in position 158 of CD16 (FCGR3A-158F homozygotes).

11. Chimeric humanised, or human monoclonal antibody composition, said composition having a concentration of over 60% for the forms G0 + G1 + G0F + G1F and a concentration for the forms G0F + G1F lower than 50%, for use in the treatment of patients homozygous for phenylalanine in position 158 of CD16 (FCGR3A-158F homozygotes) or patients heterozygous for valine/ phenylalanine in position 158 of CD16 (FCGR3A-158V/F).

12. Chimeric, humanised or human monoclonal anti-Rhesus of human red blood cell antibody composition, said composition having a concentration of over 60% for the forms G0 + G1 + G0F + G1F and a concentration for the forms G0F + G1F lower than 50%, for use in the treatment of haemolytic disease of the newborn in patients homozygous for phenylalanine in position 158 of CD16 (FCGR3A-158F homozygotes) or patients heterozygous for valine/phenylalanine in position 158 of CD16 (FCGR3A-158V/F).

13. Chimeric, humanised, or human monoclonal antibody composition targeted against a cancer cell antigen, said composition having a concentration of over 60% for the forms G0 + G1 + G0F + G1F and a concentration for the forms G0F + G1F lower than 50%, for use in the treatment of cancer in patients homozygous for phenylalanine in position 158 of CD16 (FCGR3A-158F homozygotes) or patients heterozygous for valine/ phenylalanine in position 158 of CD16 (FCGR3A-158V/F).

14. Composition according to claim 13 for use according to claim 13, **characterised in that** the antibody is an anti-HLA-DR or an anti-CD20.

15. Composition according to claim 14 for use according to claim 14, **characterised in that** the drug is intended for the treatment of cancers of HLA class-II positive cells, B-cell lymphomas, acute B-cell leukaemias, Burkitt's lymphomas, Hodgkin's lymphoma, myeloid leukaemias, B-cell chronic lymphoid leukaemias (B-CLL), T-cell leukaemias and lymphomas, non-Hodgkin's lymphomas, and chronic myeloid leukaemias.

16. Composition according to claim 13 for use according to claim 13, **characterised in that** the antibody is an antibody selected from anti Ep-CAM, anti HER2, anti CD52, anti HER1, anti GD3, anti CA125, anti GD, anti GD2, anti CD-23 and anti Protein C, anti-KIR3DL2, anti-EGFR, anti-CD25, anti-CD38, anti-CD30, anti-CD33 and anti-CD44.

17. Composition according to any of claims 11 to 16 for use according to any of claims 11 to 16, characterised that said composition has a concentration of over 80% for the forms G0 + G1 + G0F + G1F.

18. Composition according to any of claims 11 to 17 for use according to any of claims 11 to 17, **characterised in that** the forms G0F + G1F are lower than 30%.

19. Composition according to any of claims 11 to 18 for use according to any of claims 11 to 18, **characterised in that** the antibodies are IgG1.

20. Composition according to any of claims 11 to 19 for use according to any of claims 11 to 19, **characterised in that** the patients are homozygous for phenylalanine in position 158 of CD16 (FCGR3A-158F homozygotes).
